# EUROPEAN PATENT SPECIFICATION

(11) **EP 1 675 632 B1**
(45) Date of publication and mention of the grant of the patent: **12.09.2007**
(21) Application number: 04789486.0
(22) Date of filing: 30.09.2004
(51) Int. Cl.: A61M 5/31, A61M 5/28, A61M 39/20

(54) **LOW EXTRACTABLE, THERMOPLASTIC SYRINGE AND TIP CAP**
NIEDRIG EXTRAHIERBARE THERMOPLASTISCHE SPRITZE UND ENDKAPPE
SERINGUE THERMOPLASTIQUE A FAIBLES SUBSTANCES EXTRACTIBLES ET CAPUCHON POUR LA POINTE

(30) Priority: 01.10.2003 US 507137 P; 05.04.2004 US 818206
(43) Date of publication of application: 05.07.2006
(73) Proprietor: Becton, Dickinson and Company, Franklin Lakes, NJ 07417-1880 (US)
(72) Inventor: HETZLER, Kevin, Sparta, NJ 07871 (US); GROSKOPF, Roger, Saddle Brook, NJ 07663 (US)
(74) Representative: Weber, Thomas
(86) International application number: PCT/US2004/032492
(87) International publication number: WO 2005/032627

(56) References cited:
- EP-A- 0 879 611
- US-A- 5 788 670
- US-A- 5 980 495
- US-A1- 2002 133 119
- US-A1- 2002 150 644
- US-A1- 2003 171 719
- US-B1- 6 193 688

## Description

### FIELD OF THE INVENTION

The present invention is directed to a tip cap for use with a syringe where the tip cap exhibits low extractables during storage. The invention is also directed to a syringe and tip cap assembly and a prefilled syringe that is shelf stable for extended periods of time.

### BACKGROUND OF THE INVENTION

Various devices are known for transferring and storing a medicament, such as a drug or vaccine. These devices can be used for delivery of the substance to a patient. Examples of devices or containers for medicaments or vaccines include syringes, transfer sets, injection devices and vials. Medical syringes for delivering a substance to a patient include a syringe barrel with an outlet end for dispensing the substance. Typically, the syringe barrel is defined by a cylindrical wall which forms the internal chamber for containing the substance. An elongated tip extends from the outlet end and includes an axial passage that communicates with the internal chamber and the substance contained therein. The axial passage has a dimension to receive a cannula and for dispensing the substance from the syringe barrel. A plunger is generally inserted into one end of the syringe barrel. The plunger is dimensioned for forming a fluidtight seal with the inner surface of the syringe barrel and for sliding within the syringe barrel. Sliding movement of the plunger toward the outlet end and tip of the syringe barrel dispenses the substance within the internal chamber of the syringe barrel through the axial opening in the tip.

Conventional syringe barrels are often made from glass or plastic. Glass syringe barrels have the advantage of exhibiting very low gas transmissivity and are easily sterilized by conventional sterilizing techniques. Glass syringe barrels are commonly used for medications and other substances that are particularly susceptible to interaction with ambient gases. Glass syringes are also desirable for many applications since the glass syringes are generally not reactive with the substances contained therein. For these reasons, glass syringe barrels are often used for prefilled syringes where the syringe barrel is filled with a substance and stored for a considerable period of time prior to use.

Glass syringe barrels, although having many desirable properties, have several disadvantages. For example, glass syringe barrels are at a greater risk of breakage, discoloration with radiation, are more difficult to manufacture, and are heavier than other materials, which increases shipping and handling costs. Various efforts have been made to make syringes from plastic materials. Many plastic materials are easily molded so that syringe barrels can be made inexpensively. However, many plastics and plastic additives are not suitable for use in manufacturing syringes since the substance contained in the syringe can react with the plastic, thereby contaminating the substance. In addition, many of the plastics release components into the substance within the syringe during prolonged storage. These components are often referred to as extractables and render the substance unsuitable for its intended use. A particularly suitable plastic for syringe barrels is radiation stabilized polypropylene since the substance contained within a polypropylene syringe is generally shelf stable for several years.

Syringes often include a needle assembly having a needle cannula. The cannula has a first end for coupling with the syringe and an outer end with a sharpened tip. The cannula also includes a lumen that extends axially through the cannula between each of the ends. The needle assembly often includes a hub that is engageable with the tip of the syringe. The hub assembly can be coupled to the tip of the syringe so that the lumen of the cannula is in fluid communication with the axial passage through the tip of the syringe barrel. One example of a mounting device is a luer collar mounted on the tip of the syringe. The luer collar can include internal threads or tabs around the tip of the syringe that are able to mate with corresponding threads or tabs on the hub. Syringe barrels that are made of plastic are easily molded with an integrally formed luer collar. To the contrary, glass syringe barrels are not easily formed with a luer collar. Therefore, glass syringe barrels and some plastic syringe barrels include a separately molded luer collar that is mounted directly to the tip of the syringe barrel. The luer collar is typically coupled to the tip of the syringe by a snap or interference fit.

The prefilled syringe barrels containing various substances such as medications must be sealed and packaged in a manner to prevent contamination of the substance. The prefilled syringes include stoppers or closures, tip caps and tip shields over the tip of the syringe to prevent leakage, avoid contamination of the medication and to prevent the healthcare workers from unnecessary exposure to the medication. Tip caps are usually formed from an elastomeric material that is frictionally engaged with the tip of the syringe or the threaded end. The tip cap can be removed prior to use. A needle cannula hub can be securely coupled to the luer collar on the syringe barrel.

The tip caps made from an elastomeric material perform reasonably well. The resilient and flexible tip caps can, however, become separated during handling when the tip caps are frictionally engaged with the tip of the syringe barrel. The vacuum or suction effect that is created when the elastomeric tip cap is removed can result in loss or spillage of the substance and inadvertent contact of the healthcare worker with the substance.

Elastomeric materials commonly used for making tip caps are generally effective for closing and sealing the open ends of syringe barrels and cannulas. The thermoplastic elastomeric materials typically exhibit a high level of extractables which reduce the shelf life of the invention.
US-A-5,980,495 discloses that pre-filled single-use hypodermic devices must be provided with a needle cap device during storage for protection of the injector needle and the contents of the hypodermic device which is removed prior to use. The two-part needle cap device includes a plastic outer casing of hard elastic transparent material which is provided inside with a lining member sealing the tip of the injector needle and made from a soft elastic material. The injector head and the plastic outer casing have a coupling device for making a mechanical stable form-fitting connection and/or a positive-locking connection with a reliable microbiological seal when the injector head is inserted in the outer casing.

Accordingly, there is a continuing need for a syringe and a tip cap that will overcome the disadvantages and limitations of the prior devices. There is also a continuing need for an improved syringe and tip cap suitable for use in prefilled syringes.

### SUMMARY OF THE INVENTION

The present invention is directed to a tip cap for use with a syringe, a syringe assembly and a prefilled syringe. The invention is also directed to a tip cap that can be used with a prefilled syringe where the contents of the syringe are stable for extended periods of time.

Accordingly, a primary aspect of the invention is to provide a thermoplastic tip cap for use with a syringe where the tip cap exhibits low extractable levels during prolonged storage of the contents of the syringe. The thermoplastic tip cap can be produced in a sterile and clean condition.

Another aspect of the invention is to provide a tip cap that is sufficiently flexible and resilient to couple with and seal the tip of a syringe and exhibits low extractable levels to enable long term storage of the contents of a syringe. In one embodiment, the polymer composition of the tip cap and syringe barrel exhibits a residue upon ignition at 450°C-500°C of not more than 0.10 wt% based on the initial weight of the polymer composition. The weight of the residue determined according to The Japanese Pharmacopoeia, 14th Edition, No. 61, Test Methods for Plastic Containers (2001).
In particular the present invention relates to a syringe tip cap comprising: a body having a first open end, a closed second end and a bore having an internal dimension for mating with a tip of a syringe, whereby said body being made from a polymer composition comprising a thermoplastic cyclic olefin polymer or copolymer and a thermoplastic elastomer and being sufficiently flexible to couple to and seal with the tip of a syringe.

A further aspect of the invention is to provide a tip cap for coupling with a syringe where the tip cap is made from a material comprising a base thermoplastic polymer and an amount of a thermoplastic elastomer to enable the tip cap to be sufficiently flexible to couple to a tip of a syringe.

Still another aspect of the invention is to provide a tip cap made from a base thermoplastic polymer that exhibits low extractable levels, can be sterilized without loss of the flexible and resilient properties and is sufficiently flexible and resilient to couple with the tip of a syringe.

A further aspect of the invention is to provide a tip cap for coupling with a syringe where the tip cap is made from a cyclic olefin polymer and a thermoplastic elastomer, where the thermoplastic elastomer is included in an amount so that the tip cap is flexible and resilient. In one embodiment, the tip cap has a shape and dimension that is able to frictionally engage and seal the frustoconical shape tip of a syringe. In another embodiment, the tip cap has a shape to mate with a threaded tip or collar and can have internal or external threads.

Another aspect of the invention is to provide a tip cap for use with a prefilled syringe where the polymer composition of the tip cap exhibits low extractable levels as determined by a 20 ml sample test solution having a residue of not more than 1.0 mg after evaporation and drying for 1 hour at 105°C, where the sample test solution is obtained by autoclaving the polymer composition in 200 ml of water at 121°C for 1 hour. The polymer composition also preferably exhibits a residue upon ignition at 450°-500°C of not more than 0.10 wt% based on the initial weight of the polymer composition. The weight of the residue of extractables and the residue upon ignition are determined by The Japanese Pharmacopoeia, 14th Edition, No. 61, Test Methods for Plastic Containers (2001). In preferred embodiments, the complete syringe assembly including the syringe barrel, tip cap, and stopper and plunger meet these requirements.

A further aspect of the invention is to provide a flexible and resilient tip cap for sealing a syringe tip, where the tip cap remains flexible and resilient after sterilization by gamma radiation, ethylene oxide treatment and steam autoclave.

These and other aspects of the invention are basically attained by providing a syringe tip cap comprising: a body having a first open end, a closed second end and a bore, where the bore has an internal dimension for mating with a tip of a syringe. The body is made from a polymer composition comprising a thermoplastic cyclic olefin polymer or copolymer and a thermoplastic elastomer and is sufficiently flexible to couple to and seal with the tip of a syringe.

The aspects of the invention are also attained by providing a prefillable, low particle, low extractable syringe comprising: a syringe assembly including a syringe barrel having a substance receiving chamber and a tip extending from a distal end of the syringe barrel and having a fluid passage extending through the tip, and a plunger received in the syringe barrel. A tip cap is coupled to the tip for closing the tip. The tip cap is sufficiently flexible and resilient to be removably coupled to the tip and form a substantially fluidtight seal with the tip. The tip cap is made from an elastic and flexible polymer composition comprising a thermoplastic cyclic olefin polymer or copolymer and a thermoplastic elastomer.

The various aspects of the invention are further attained by providing a prefilled syringe comprising a syringe barrel having a hollow body with a first open end and a tip with a bore extending into the hollow body. A plunger is received in and closes the first open end. A solution or suspension is contained within the syringe barrel to define the prefilled syringe. A tip cap is removably coupled to the tip. The tip cap is made from a flexible and resilient polymer composition comprising a thermoplastic cyclic olefin polymer or copolymer and a thermoplastic elastomer.

These and other aspects of the invention will become apparent from the drawings and the detailed description of the invention which disclose various embodiments of the invention.

### BRIEF DESCRIPTION OF THE DRAWINGS

Referring to the drawings, which form a part of this original disclosure, in which:

Figure 1 is an exploded perspective view of the syringe and tip cap assembly in one embodiment of the invention;

Figure 2 and Figure 2A are partial cross-sectional views of the tip cap in embodiments of the invention; and

Figure 3 is a cross-sectional side view of a prefilled syringe in another embodiment showing the syringe, cannula and tip cap.

### DETAILED DESCRIPTION OF THE INVENTION

The present invention is directed to a tip cap, a syringe and tip assembly and a prefilled syringe. In particular, the invention is directed to a flexible and resilient member for use with a prefilled syringe where the substance contained in the syringe is stable for prolonged periods of time. In one embodiment, the invention is directed to a clean and sterile syringe barrel, plunger and tip cap assembly. The syringe barrel can be filled for immediate use or filled for subsequent distribution as a prefilled syringe.

Prefilled syringes is a term of art for syringes that are filled with a substance, such as a medication, by the manufacturer or supplier and shipped to the healthcare provider for use without further treatment. The prefilled syringes are packaged in a clean and sterile condition that are ready for use by the healthcare provider without the need to prepare the syringe for use. Prefilled syringes are intended for single use and intended to be disposable. Prefilled syringes are made of various materials that do not interfere with the substance contained in the syringe so that the substance is stable for about 6 months or more when refrigerated or stored at room temperature. In preferred embodiments of the invention, the substance contained in the prefilled syringe is stable for at least 6 months, and preferably 3-5 years, when stored at temperatures of 15° to 30°C.

The stability of a substance contained in a prefilled syringe is determined by analysis of the substance after storage for a prolonged period of time. It is known that various components and additives from the plastic material used to manufacture the syringe and other containers or components of the syringe assembly can leach from the plastic and contaminate the substance within the syringe. These impurities can be additives that are combined with the plastic resin to provide various properties during the molding process or properties in the finished product. Examples of additives that can result in impurities in the substance within the syringe or container after storage include extrusion processing agents, antioxidants, nucleating agents, UV absorbing agents and clarifying agents.

In preferred embodiments of the invention, the syringe assembly including the syringe barrel, stopper and tip cap, and the prefilled syringe exhibit substantially no dissolution of materials or components into the substance within the syringe. The resulting impurities from the plastic materials in the contents of the syringe are referred to as extractables. After storage, the substance in the prefilled syringe is substantially free of visible components or extractables and free of contamination. In one embodiment, the substance in the prefilled syringe contains impurities in amounts of about 0.1 ppm or less after storage for 6 months at room temperature.

In preferred embodiments, the syringe assembly including the tip cap, the syringe barrel and stopper before and after sterilization by gamma radiation, ethylene oxide and autoclaving meet or exceed the standards for extractable substances as determined by The Japanese Pharmacopoeia, 14th Edition, No. 61, Test Methods for Plastic Containers (2001). In another embodiment, the syringe assembly and the respective component before and after sterilization satisfy the standards of The Japanese Pharmacopoeia, 14^{th} Edition, No. 59, *Test for Rubber Closure for Aqueous Infusions*.

In one embodiment, the polymer composition of the tip cap and syringe satisfies the combustion tests of No. 61, Test Methods for Plastic Containers of The Japanese Pharmacopoeia (2001). The combustion test is based on the weight of the residue upon ignition and the measured amounts of lead, cadmium and tin. The residue on ignition is determined by placing 5 g of the polymer composition in a crucible and heating between 450°C-500°C. The weight of the residue on ignition of not more than 0.10 wt% is considered as passing the residue on ignition test. In one preferred embodiment, the polymer composition of the invention passes this test by producing a residue on ignition of not more than 0.10 wt% after heating between 450° and 500°C based on the weight of polymer composition. The test method for lead according to The Japanese Pharmacopoeia uses 2.0 g of the polymer composition moistened with 2 ml of sulfuric acid which is heated in a crucible between 450°C and 500°C. The residue is moistened with water and 2-4 ml of HCl and evaporated to dryness. Then 1-5 ml of hydrochloric acid as added and warmed to dissolve the residue. Then 0.5-1 ml of a mixture of citric acid monohydrate and HCl were added with 0.5-1 ml of a mixture of citric acid monohydrate and HCl were added with 0.5-1 ml of warm ammonium acetate. The solution is filtered and combined with 10 ml of diammonium hydrogen citrate. Bromothymol and ammonia are added until the solution changes color. Ammonium sulfate solution is then added and the solution diluted with water. N,N-diethyldithiocarbamate trihydrate and 4-methyl-2-pentanone were added, shaken and the organic layer separated. The resulting test sample was tested by atomic absorption spectrophotometry using a lead hollow-cathode lamp at 283.3 nm. The polymer composition is considered to pass this test when the absorbance is not more than half of a standard lead solution.

The test for cadmium was conducted in the same way using a cadmium hollow-cathode lamp at 228.8 nm. The polymer composition is considered to pass this test when the absorbance is not more than the absorbance of a standard cadmium solution. In one embodiment of the invention, the polymer composition has 200 ppb or less of lead, 20 ppb or less of cadmium and 100 ppb or less of tin.

In one embodiment of the invention, the polymer composition of the syringe and tip cap after sterilization satisfies the acceptable limits for extractable substances as defined by the foaming test, pH test, potassium permanganate-reducing substances test, UV spectrum test and residue on evaporation test according to The Japanese Pharmacopoeia, No. 61, Test Methods for Plastic Containers (2001). Each of the tests are based on a sample solution prepared by cutting pieces of the polymer composition into strips 0.5 cm wide and 5 cm long to provide a total surface area of 1200 cm² when the thickness of the polymer composition is less than 0.5 mm or 600 cm² when the thickness is greater than 0.5 mm. The strips are washed with water, dried and heated in 200 ml water in an autoclave at 121°C for 1 hour. The resulting sample test solution is cooled to room temperature. A blank solution is prepared in the same way.

The foam test is performed using 5 ml of the test solution in a 15 mm by 200 mm tube and shaken vigorously for 3 minutes. The polymer composition satisfies the foam test when the foam almost disappears within 3 minutes. In one embodiment, the polymer composition after sterilization satisfies this test by producing a foam that disappears in less than 3 minutes.

The pH test adds 1.0 ml of a potassium chloride solution (1 in 1000) to 20 ml of the test solution and the blank solution and measuring the pH. The polymer composition satisfies the pH test when the difference between the pH of the test solution and the blank is not more than 1.5.

The test for potassium permanganate-reducing substances adds 20.0 ml of 0.002 mol/L potassium permanganate VS and 1 ml of dilute sulfuric acid to 20 ml of the test solution and boiling for 3 minutes. After cooling 0.10 g potassium iodide is added, shaken and allowed to stand for 10 minutes. The resulting solution is titrated with 0-01 mol/L sodium thiosulfate VS (indicator:5 drops of starch TS).. The blank solution is treated in the same way using 20.0 ml of the blank solution. The difference in the consumption of 0.002 mol/L potassium permanganate VS is measured. The polymer composition after sterilization satisfies this test by exhibiting a consumption of the potassium permanganate solution for the test solution of not more than 1.0 ml than the blank solution.

The UV spectrum is performed by reading the maximum absorbencies. The polymer composition after sterilization satisfies this test by the differences between the maximum absorbency of the test solution and blank solution between 220 nm and 240 nm being not more than 0.08 and between 241 and 350 nm being not more than 0.05. The polymer composition of the syringe barrel and tip cap after sterilization satisfies the residue on evaporation test when a 20 ml test solution of the polymer composition produces a residue of not more than 1.0 mg after evaporation and drying for 1 hour at 105°C, where the sample test solution is obtained by autoclaving the polymer composition in 200 ml of water at 121 °C for 1 hour.

The polymer composition of the tip cap and the syringe barrel also satisfy the leakage test and cytotoxicity test of No. 61, *Test Methods for Plastic Containers.* The leakage test is performed by filling the syringe assembly with the associated syringe barrel, tip cap and stopper with a sodium fluoroscein solution, placing the syringe barrel on a filter paper and applying a pressure of 6.9 N at 20°C for 10 minutes. Leakage is determined by observing the color of the filter paper. In one embodiment of the invention, the syringe barrel exhibits no leakage by this test. The cytotoxicity is determined by evaluating culture medium extracts where the IC₅₀ (%) is not less than 90%.

In another embodiment, the polymer composition has a low extractable content as determined by a sample solution having a transparency of not less than 99.0% at 430 nm and 650 nm where the sample solution is obtained by autoclaving the polymer composition in 10 times the amount of water by weight at 121°C for 1 hour as described in The Japanese Pharmacopoeia, 14^{th} Edition, No. 59, *Test for Rubber Closure for Aqueous Infusions.* Preferably, the extractable content is sufficiently low such that the resulting sample solution is clear and colorless. The polymer composition also exhibits low extractable substances as determined a residue on evaporation of 100 ml of the sample solution of not more than 2.0 mg where the sample solution is evaporated to dryness and the residue dried at 105°C for 1 hour. The extractable substances content of the polymer composition is low as determined by the sample solution having an absorbance of not more than 0.20 by ultraviolet-visible spectrophotometry against a blank solution. Each of these measurements for extractable substances is determined from a sample solution obtained by autoclaving the polymer composition in 10 times the amount of water by weight at a temperature of 121°C for I hour as described in The Japanese Pharmacopoeia, 14^{th} Edition, No. 59.

In another embodiment, the polymer composition also meets or exceeds the requirements for extractable substances according to the foam test as described in The Japanese Pharmacopoeia, 14^{th} Edition, No. 59. A 15 ml amount of the sample test solution as prepared above for extractable substances is placed in a 15 mm diameter by 200 mm long tube is shaken vigorously for 3 minutes. The extractable substance content is sufficiently low that the resulting foams disappear in 3 minutes. The extractable substance content is also sufficiently low as determined by adding 1.0 ml of a potassium chloride solution (1.0 g/L potassium chloride) in 20 ml of the sample test solution such that the difference between the pH of the sample test solution and a blank solution is not more than 1.0.

The polymer composition typically also has low extractable substances as determined by a sample test solution having an absorbance by atomic absorption spectrophotometry that is not more than the absorbance of a standard zinc solution at 213 .9 nm as described in The Japanese Pharmacopoeia, 14^{th} Edition, No. 59. The sample test solution is added in an amount of 10 ml with nitric acid to make 20 ml. The standard zinc solution is prepared from 10 ml of a standard zinc stock solution and water to make 10O0 ml so that 1 ml of this solution contains 0.01 mg zinc. The standard zinc stock solution is produced by dissolving 1 g of zinc in 100 ml water and 5 ml hydrochloric acid.

The polymer composition of the tip cap and syringe also meet or exceed the standards for impurities and extractables under the U.S. and European requirements for medical devices and containers. The U.S. standard is based on the guidelines set forth in USP <661> according to the U.S. Pharmacopoeia. This test measures the leaching from plastic using 120 cm² of the plastic in 20 ml purified water at 70°C for 24 hours.

The polymer composition of the tip cap and syringe does not interact physically or chemically with the substance in the syringe to alter any property or quality and does not permit the invasion of microbes. The polymer composition preferably meets or exceeds the absorbance of a sample solution in a cadmium hollow-cathode lamp, the absorbance of a solution in a lead hellow-cathode lamp and the tests for acute systemic toxicity as determined according to The Japanese Pharmacopoeia, 14^{th} Edition, No. 59, *Test for Rubber Closure for Aqueous Infusion*.

The absorbance in a cadmium hollow-cathode lamp according to The Japanese Pharmacopoeia, 14^{th} Edition, No. 59, is determined by incinerating a sample of the polymeric composition, and dissolving the residue in hydrochloric acid and ammonium acetate. Tetra sodium bromomethyl blue and tetrasodium ammonium are added to the solution to obtain a color change followed by the addition of ammonium sulfate, sodium N,N,diethyldithiocarbamate trihydrate and 4-methyl-2-pentanone. The standard test solution prepared from a standard calcium solution containing 0.1 mg calcium per 1 ml and adds diammonium hydrogen citrate and bromothymol blue. The absorbance of the sample solution is measured against the standard test solution with a cadmium hollow-cathode lamp at 228.8 by atomic absorption spectrophotometry where the absorbance of the sample solution is not more than the standard.

The absorbance of sample solution is also measured using a standard lead solution with a lead-hollow-cathode lamp at 283.3 nm. The standard test solution is produced by adding hydrogen citrate and bromothymol blue to a standard lead solution containing 0.1 mg lead per 1 ml.

In one embodiment, the syringe barrel, stopper and tip cap are cleaned and sterilized and then filled with a desired substance to be delivered to a patient. The syringe assembly can be sold or distributed to a supplier such as a pharmaceutical company where the syringe is subsequently filled. The syringe is again sterilized such as by autoclaving either before or after filling the syringe. The tip cap of the invention remains sufficiently flexible and resilient after multiple sterilization steps to maintain an effective seal, structural integrity, and exhibits low extractable levels.

The tip cap of the invention is also flexible and resilient to enable sealing and coupling to the tip of the syringe. The tip cap also has sufficient strength to withstand the torque removal force that is necessary to remove the tip cap from the syringe by a twisting motion when the tip cap is coupled to the tip of a syringe in a manner that a fluidtight seal is formed. The tip cap also has sufficient strength to overcome the suction that occurs when the tip cap is pulled from the syringe tip. The tip cap of the invention is preferably made from a polymer composition that remains flexible and resilient after sterilization. The tip cap can be sterilized by autoclaving, radiation and gas sterilization, such as by ethylene oxide, and remains flexible and exhibits low extractable levels after sterilization.

In preferred embodiments of the invention, the tip cap is made from a polymer composition comprising a base resin and a thermoplastic elastomer. The base resin provides sufficient strength for the tip cap and preferably satisfies the test requirements and standards for extractable components after sterilization. The polymer composition can include various additives provided the additives are in amounts that do not react with the substance contained in the prefilled syringe and do not increase the level of extractables above the acceptable levels after sterilization. Examples of additives that can be used include plasticizers, ultraviolet absorbers, thermal stabilizers, antioxidants and antistatic agents.

In preferred embodiments, the base resin is a cyclic olefin polymer or copolymer made from a cyclic unsaturated monomer. The cyclic olefin polymers and copolymers are particularly suitable since these materials withstand sterilization as required for medical devices and exhibit very low extractable levels within acceptable limits for medical devices and containers. In one embodiment, the base resin consists essentially of a cyclic olefin copolymer.

Preferred cyclic olefin copolymers are ZEONEX and ZEONOR from Nippon Zeon Company Ltd. Examples of other cyclic olefin copolymers that can be used include those available under the trademark TOPAS by Hoechst AG and APEL of Mitsui Petrochemical Industries Ltd.

In one preferred embodiment, the cyclic olefin polymer is a norbomene polymer or copolymer having the structure

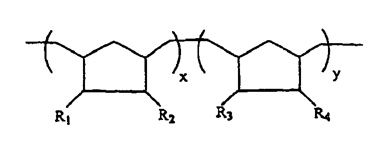

where R₁, R₂, R₃ and R₄ are independently selected from the group consisting of H, alkyl, alkoxyl, cyano, alkoxycarbonyl, phenyl, and substituted phenyl and x and y are integers.

In one embodiment, the polymers and copolymers are made from the polymerization of norbomene by the ring-opening metathesis polymerization (ROMP). The copolymers can be random or block copolymers. In other embodiments, the cyclic olefin copolymer can be made by vinyl-type or cationic or radical type polymerization. Examples of norbornene monomers include 2-norbornene, 5-methyl-2-norbornene, 5,5-dimethyl-2-norbornene, 5-ethyl-2-norbomene, 5-butyl-2-norbornene, 5-ethylidene-2-norbornene, 5-methoxycarbonyl-2-norbornene, 5-cyano-2-norbornene, 5-methyl-5-methoxycarbonyl-2-norbornene, 5-phenyl-2-norbornene, 5-phenyl-5-methyl-2-norbornene, 5-hexyl-2-norbornene, 5-octyl-2-norbornene, and 5-octadecyl-2-norbornene.

Cyclic olefin copolymers are available from Nippon Zeon Co. Ltd. Examples of other cyclic olefin copolymers are disclosed in U.S. -A- 5,561,208.

The TOPAS cyclic olefin copolymers can be produced by conventional polymerization processes from cyclic monomers having at least one unsaturated double bond and copolymerizing the cyclic olefin monomer with at least one other ethylenically unsaturated monomer. Suitable cyclic olefin monomers can be monocyclic or polycyclic. Examples of suitable monocyclic monomers include cyclohexene and cyclopentene. Examples of particularly suitable polycyclic unsaturated monomers include norbornene and tetracyclododecene, which can be substituted with one or more substituents. For example, the cyclic olefin monomers can be substituted with one or more hydrocarbon radicals such as a C₁-C₆- aryl radical and a C₁-C₈ alkyl radical. In further embodiments, the polycyclic monomer can be substituted with an alkyl such as methyl, ethyl, propyl, isopropyl, amyl, hexyl, octyl, decyl, dodecyl and octadecyl. The aryl substituents can include phenyl, tolyl, naphthyl, benzyl and phenylethyl groups.

The cyclic olefin monomer can be copolymerized with an α-olefin having at least 2 carbons and can be straight chain or branched. The α-olefin can have 2-20 carbon atoms. Examples of non-cyclic olefin monomers suitable for copolymerizing with the cyclic olefin monomers includes ethylene, propylene, 1-butene, 1-pentene, 1-hexene, 3-methyl-1-butene, 3-methyl-1-pentene, 3-ethyl-1-pentene, 4-methyl-1-pentene, 4-methyl-1-hexene, 4,4-dimethyl-1-hexene, 4,4-dimethyl-1-pentene, 4-ethyl-1-hexene, 3-ethyl-1-hexene, 1-octene, 1-decene, 1-dodecene, 1-tetradecene, 1-hexadecene, 1-octadecene and 1-eicosene. Ethylene and propylene are generally preferred.

The thermoplastic elastomer is preferably non-halogenated and free or substantially free of components that can disperse into the substance contained in the prefilled syringe. The thermoplastic elastomer is able to be dispersed in and blended with the base polymer in amounts such that the resulting polymer composition is flexible and resilient to form a seal with the syringe. The thermoplastic elastomer is selected to provide the necessary flexibility and resilience and to be sufficiently compatible with the syringe to enable the tip cap to be separated from the syringe. In particular, the thermoplastic elastomer is used in amounts and is selected so that the tip cap does not adhere to the syringe to such an extent that the tip cap cannot be readily separated. In the embodiments where the syringe is made from a thermoplastic resin, the tip cap is made with a polymer blend that does not permanently self-adhere to the syringe.

The thermoplastic elastomer has rubber-like properties to provide the desired flexibility and resilience to the tip cap. Preferably, the thermoplastic elastomer is a block copolymer and has a glass transition temperature of 40°C or lower. Examples of suitable thermoplastic elastomers are block copolymers of aromatic vinyl monomers with a conjugated diene monomer, random copolymers of an aromatic vinyl monomer with a conjugated diene monomer and hydrogenation products thereof and norbornene-based elastomers. Particularly preferred thermoplastic elastomers are selected from the group consisting of styrene-butadiene block copolymers, styrene-butadiene-styrene block copolymers, styrene-isoprene block copolymers, styrene-isoprene-styrene block copolymers and hydrogenated products thereof and styrene-butadiene random copolymers. In one embodiment of the invention, the thermoplastic elastomer is a styrene-ethylene-ethylene-propylene-styrene block copolymer. A particularly preferred thermoplastic elastomer is available from Kuraray Co. Ltd. under the tradename SEPTON 4055.

Examples of suitable thermoplastic elastomers include random or block styrene-butadiene copolymer, such as styrene-butadiene rubber and high styrene rubber, and the hydrogenation products thereof; isoprene rubber and the hydrogenation products thereof; chloroprene rubber and the hydrogenation products thereof; saturated polyolefin rubber, such as ethylene-propylene copolymer, ethylene-α-olefin copolymer, and propylene-α-olefin copolymer; diene-containing polymers, such as ethylenepropylene-diene copolymer, α-olefin-diene copolymer, diene copolymer, isobutylene-isomer copolymer, and isobutylenediene copolymer, and the hydrogenation products of the diene-containing polymers, and acrylonitrile-butadiene copolymers and their hydrogenation products.

Other thermoplastic elastomers include epichlorohydrin rubber, propylene oxide rubber, and ethylene-acrylic rubber. Examples of norbornene-based thermoplastic elastomers include copolymers of a norbornene-group monomer with ethylene or α-olefin, and terpolymers of a norbornene-group monomer, ethylene and α-olefin, ring-opening polymers of a norbornene-group monomer, and the hydrogenation products of ring-opening polymers of a norbornene-group monomer; random copolymers of an aromatic vinyl monomer with a conjugated diene, such as styrene-butadiene-styrene rubber, styrene-isoprene-styrene rubber, and styrene-ethylene-butadienestyrene rubber, and the hydrogenation products thereof; thermoplastic elastomers, which include styrene-based thermoplastic elastomers, for example, linear or radial block copolymers of an aromatic vinyl monomer and a conjugated diene, such as styrene-butadiene-styrene rubber, styrene-isoprene-styrene rubber, and styrene-ethylene-butadiene-styrene rubber, and the hydrogenation products of them, and further, urethane-based thermoplastic elastomers, polyamide-based thermoplastic elastomers, polyamide-based thermoplastic elastomers, 1,2-polybutadiene-based thermoplastic elastomers, poly(vinyl chloride)-based thermoplastics elastomers, and fluorine-containing thermoplastic elastomers. As further examples, there may be mentioned such high molecular compounds as polyacrylic or polymethacrylic resins having a cyclic substituent, such as the cyclohexyl group, isobornyl group, tricyclo[4.3.0.1^{2.5}]-decane-3-yl group and tricyclo[4.3.0.1^{2.5}]7-decen-3-yl group; copolymers of styrene with an acrylate or methacrylate, such as octyl acrylate, hexyl acrylate, and butyl acrylate; polyamide resins, such as poly(amino-carbonyltetramethylenecarbonylaminomethylene-1,3-cyclohexylenem ethylene); polyester resins such as poly[oxycarbonyl(1,3-phenylene)carbonyloxymethylene(tricyclo [4.3.0.1^{2.5}]-3,8-diyl)methylene]; polyether resins such as poly(butylene oxide), poly[oxy(2-methyl-2-hydroxytrimethylene)oxy(1,4-phenylene)isopropylidene(1 ,4-phenylene)]; polycarbonate resins such as poly[oxycarbonyloxy(2-methyl-1,4-cyclohexylene]isopropylidene(3-methyl-1,4 -cyclohexylene)]; and polyurethane resins.

In one embodiment, the thermoplastic elastomers are copolymers of an aromatic vinyl monomer with a conjugated diene type monomer, the hydrogenation products thereof, and norbornene-based elastomers since they exhibit good dispersibility in the thermoplastic norbornene polymers when norbomene polymers are used as the base resin. The copolymers of an aromatic vinyl monomer with a conjugated diene type monomer may be either a block copolymer or a random copolymer. Polymers having unsaturated groups other than aromatic rings that have been hydrogenated are also suitable. Specific examples of such polymers include styrene-butadiene block copolymer, styrene-butadiene-styrene block copolymer, styrene-isoprene block copolymer, styrene-isoprene-styrene block copolymer, the hydrogenation products thereof, and styrene-butadiene random copolymer.

In preferred embodiments, the thermoplastic elastomer is a non-halogenated elastomer to avoid the dispersion of chlorine-containing compounds and impurities into the substance contained in the prefilled syringe after prolonged storage. Where the halogenated compounds and the associated risk of contamination is not of concern, various halogenated elastomers can be used. Examples of halogenated elastomers include; vinylidene fluoride-ethylene trifluoride copolymer, vinylidene fluoride-propylene hexafluoride copolymer, vinylidene fluoride-propylene hexafluoride-ethylene tetrafluoride copolymer, propylene-ethylene tetrafluoride copolymer; and chlorosulfonated polyethylene rubber.

In other embodiments, the elastomer is an isobutylene based copolymer. The isobutylene based copolymer can be halogenated and is typically a highly branched. Examples of butylene based copolymers includes butyl rubber, polyisobutylene and copolymers of C₄-C₇ isomonoolefin and a para-alkylstyrene. Examples of thermoplastic elastomeric copolymers are disclosed in U.S. -A- 5,654,379, 5,548,029 and 5,548,012.

Cyclic olefin copolymers are particularly preferred for producing the tip cap of the invention. The cyclic olefin copolymers have an amorphous structure and are typically clear transparent materials. In addition, the cyclic olefin copolymers can be sterilized by gamma radiation, steam autoclaving or ethylene oxide gas without altering the physical properties of the copolymer. The copolymers also exhibit low shrinkage, low distortion and low warpage after sterilization.

The cyclic olefin copolymer is blended with at least one thermoplastic elastomer in an amount to form a polymer resin composition that is flexible and resilient. Cyclic olefin copolymers are typically hard and stiff and are not able to form an effective fluidtight seal. A tip cap produced from a cyclic olefin copolymer alone is rigid and hard and is not easily coupled to a conical shaped tip of a syringe and is not able to form a fluidtight seal. The hard and rigid cyclic olefin is also not able to couple with a luer fitting and form a reliable fluidtight seal. By blending a thermoplastic elastomer with the cyclic olefin copolymer, the flexibility and resilience of the blend can be selected so that the resulting tip cap is able to form a leakproof seal and is able to couple with the tip of a syringe. Preferably, the thermoplastic elastomer is selected to be compatible with the polymer that is used to form the syringe so that the tip cap is able to form a fluidtight seal without permanently adhering to the syringe.

The amount of the thermoplastic elastomer blended with the cyclic olefin copolymer can vary depending on the elastomer and the desired flexibility of the blend. Typically, the thermoplastic elastomer is blended with the cyclic olefin copolymer in an amount of 20 wt% to 50 wt% based on the total weight of the polymer resin composition. In preferred embodiments, the polymer resin composition includes the thermoplastic elastomer in an amount of 30 wt% to 40 wt%, and more preferably 30 wt% based on the total weight of the polymer resin composition. In one embodiment, the polymer composition comprises 50 wt% to 80 wt% norbornene thermoplastic copolymer and 20 wt% to 50 wt% of a thermoplastic elastomer.

It has been found that increasing the amount of the elastomer that is blended with the cyclic olefin copolymer decreases the flexural modulus and tensile modulus of the resin composition. In one embodiment, the tip cap can have a flexural modulus similar to polypropylene. A resin composition containing 45 wt% of a thermoplastic elastomer has a flexural modulus of 755MPa (120,000 psi) and a flexural modulus that is substantially equivalent to polypropylene. A resin composition containing 40-42.5 wt% of a thermoplastic elastomer has a flexural modulus of 824MPa (110,00 psi).

The thermoplastic elastomer can be any suitable polymer that is compatible with the cyclic olefin copolymer and is able to provide the desired amount of flexibility and resilience to form a seal. The thermoplastic elastomer preferably is used in amounts that do not result in high extractable levels after sterilization and do not contaminate the contents of the syringe.

In one embodiment, the polymer resin composition includes a polymer component that consists essentially of a thermoplastic cyclic olefin copolymer and a thermoplastic elastomer. The polymer component of the polymer resin composition can consist essentially of 20 wt% to 50 wt% of a thermoplastic elastomer and 50 wt% to 80 wt% of a thermoplastic cyclic olefin copolymer. In another embodiment, the polymer component of the polymer resin composition consists essentially of a norbomene copolymer and a styrene-ethylene-ethylene-propylene-styrene block copolymer thermoplastic elastomer.

The thermoplastic cyclic olefin copolymer and the thermoplastic elastomer can be blended using various known processes. The suitable mixing processes include kneading, roller millers, single screw extruder, twin screw extruder, continuous melt mixer, Branburry mixer and other types of paddle-type mixers. In one embodiment, the cyclic olefin copolymer and the thermoplastic elastomer are blended to uniformly disperse the thermoplastic elastomer in the thermoplastic cyclic olefin copolymer. The resulting mixture is then fed to an extruder where the components are melt-blended and injection molded. The resulting polymer composition is injection molded to form the tip cap assembly using standard injection molding processes and equipment.

The elastomer is preferably a thermoplastic resin that can be blended with the cyclic olefin copolymer without adversely affecting the processability and molding of the resulting polymer composition. The elastomer is preferably stable at steam sterilizing temperatures and is able to withstand sterilization by ethylene oxide and gamma radiation treatment.

The syringe can be made of various materials that satisfy the requirements for extractables and stability of the substance contained within the syringe. Suitable materials for the syringe include glass and polypropylene. In one embodiment of the invention, the syringe is injection molded from a cyclic olefin copolymer. Preferred cyclic olefin copolymers are norbornene cyclic olefin copolymers as previously discussed. The cyclic olefin copolymer of the syringe can be the same or different than the cyclic olefin of the tip cap. The polymer resin composition that is used for the syringe and the tip cap are selected such that the tip cap can form an effective fluidtight seal and can be easily removed from the syringe.

Referring to the drawings, a hypodermic syringe assembly 10 includes a syringe barrel 12 and a tip cap 14. Syringe barrel 12 is made from a suitable material for medical use. In preferred embodiments, syringe barrel 12 is made from a material that is suitable for producing prefilled syringes where the substance contained in the syringe is stable for extended periods of time. Examples of suitable materials include glass and various thermoplastics. In preferred embodiments, the syringe barrel is made from a cyclic olefin copolymer. In other embodiments, thermoplastics, such as polypropylene, can be used.

Syringe barrel 12, in the embodiment illustrated, has a substantially cylindrical configuration with a side wall 16 extending from a first end 18 to an outlet end 20. Cylindrical side wall 16 defines a fluid receiving chamber 22 having an internal dimension sufficient to contain a desired amount of a substance to be delivered to a patient. The outlet end 20 of syringe barrel 12 has an axially extending tip 24. Tip 24 is integrally formed with syringe barrel 12. Tip 24 is formed with an internal passage 26 extending axially through tip 24 and being in communication with chamber 22 for dispensing the contents of syringe barrel 12. In the embodiment illustrated, tip 24 has a substantially frustoconical shape that converges from an end wall 28 of syringe barrel 12 toward the outlet end 20.

In the embodiment illustrated, a luer collar 34 is integrally molded with syringe barrel 12 and surrounds tip 24. Luer collar 34 includes a cylindrical side wall 36 and an open end 40. In an alternative embodiment, luer collar 34 can be a separate collar that is coupled to the tip 24 of syringe barrel 12. The separate luer collar is coupled to tip 24 by sliding tip 24 through the opening in an end wall to snap the collar onto the tip. Side wall 36 of luer collar 34 includes internal threads 42 for coupling with a cannula or other fitting. In other embodiments, coupling tabs can be provided in place of the threads for coupling with a suitable fitting.

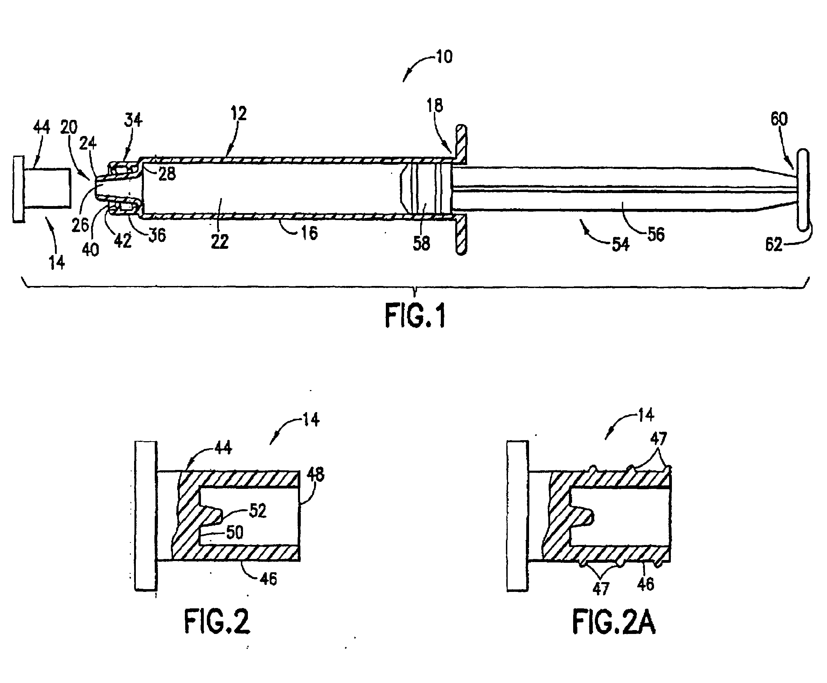

Tip cap 14 is made from a flexible and resilient material that is able to couple to tip 24 or luer collar 34 and form a fluidtight seal to prevent leakage of the contents of syringe barrel 12. As shown in Figure 2, tip cap 14 includes a body portion 44 having a cylindrical side wall 46 defining an axial bore and forming an open end 48. Cylindrical side wall 46 and open end 48 have an inner surface with an internal dimension capable of mating with tip 24. In the embodiment illustrated, body 44 of tip cap 14 has a closed bottom end 50 with a projection 52 defining a stopper for tip 24. Projection 52 is oriented within bottom end 50 of body 44 and has a dimension to be inserted into axial passage 26 of tip 24 when tip cap 14 is positioned on tip 24. In one embodiment shown in Figure 2A, tip cap 14 can include external threads 47 on cylindrical side wall 46 for coupling with the internal threads of luer collar 34. Alternatively, tip cap 14 can have internal threads for coupling with threads on the syringe. In preferred embodiments, tip cap 14 is injection molded from a resilient and flexible polymer composition so that the inner surface of cylindrical side wall 46 is able to couple to and seal the frustoconical shaped tip 24 of the syringe. Preferably, the tip cap is made from a resin composition having a polymer component that includes a blend of a thermoplastic cyclic olefin copolymer and a thermoplastic elastomer.

In preferred embodiments, syringe assembly 10 is a prefillable syringe that can be prefilled with a substance such as a drug to be delivered to the patient. Typically, syringe barrel 12 and tip cap 14 are assembled and the assembly is sterilized and cleaned prior to filling by standard sterilizing methods used for sterilizing medical devices. Sterilizing is generally performed by treating syringe barrel 12 and tip cap 14 with ethylene oxide or gamma radiation or combinations thereof. Other suitable sterilizing processes include steam autoclaving, treating with hydrogen peroxide, or an ozone and steam mixture. X-rays, neutron beams or beta beams, and mixtures thereof, can also be used to sterilize syringe barrel and tip cap 14. In preferred embodiments, syringe barrel 12 and tip cap 14 are made from materials that do not .contaminate the contents of the prefilled syringe, such that the contents are stable when stored for extended periods of time even after one or more sterilization processes. Preferably, the contents are stable for at least six months, and more preferably 3-5 years, when stored at room temperature with substantially no contamination of the contents after sterilization. The syringe assembly is suitable for prefilled syringes containing, for example, a contrast media for radiography, MRI and ultrasonography. The syringe assembly can also be prefilled with a drug or medication solution.

In the embodiment of Figure 1, syringe barrel 12 is shown with an integral luer collar that surrounds tip 24. In further embodiments, syringe barrel 12 can be produced with separate luer collar. Injection molded syringe barrels made from thermoplastics are readily formed with integrally formed luer collars by standard injection molding processes. Injection molded syringes for producing prefilled syringes can be made from polypropylene and other polymers that do not affect the long term stability of the substance contained within the prefilled syringe. In one embodiment of the invention, the prefilled syringe is made from a cyclic olefin copolymer.

Syringe assembly 10 includes a plunger assembly 54 including a plunger rod 56 and a plunger 58 coupled to one end of plunger rod 56. Plunger 58 is formed from a flexible rubber-like material capable of forming a seal with the inner surface of cylindrical side wall 16 and sliding axially within syringe barrel 12 to dispense the contents of the syringe. In one embodiment, plunger 58 can be made from the same material as tip cap 14. Alternatively, plunger 58 can be made from the same polymer composition that is used to make the tip cap 14. Plunger rod 56 includes an outer end 60 having a flange 62 for operating plunger assembly 56.

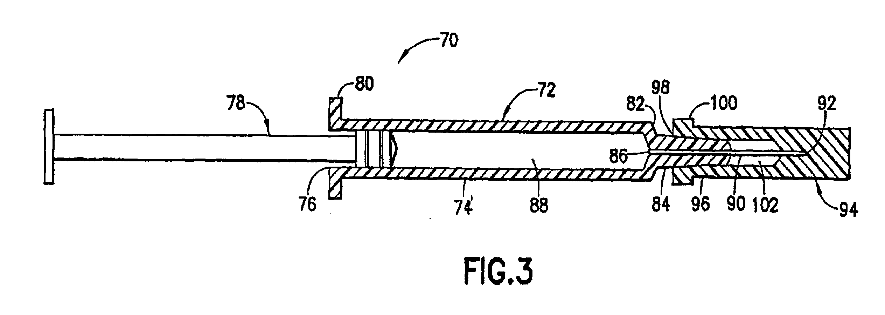

Referring to Figure 3, a second embodiment of a prefilled syringe is shown. Prefilled syringe assembly 70 includes a syringe barrel 72 having a cylindrical side wall 74. Side wall 74 has an open end 76 for receiving a plunger assembly 78. A flange 80 extends radially outward from open end 76 of side wall 74.

Syringe barrel 72 has an outlet end 82 converging toward a conical shaped tip 84. Tip 84 includes an axial passage 86 in communication with the internal chamber 88 defined by cylindrical side wall 74.

In the embodiment of Figure 3, a needle cannula 90 is coupled to tip 84 and positioned within axial passage 86. Cannula 90 also includes an axial passage in fluid communication with chamber 88 and terminates at a distal end having a sharpened tip 92.

A tip cap 94 is coupled to tip 84 of syringe assembly 70 as shown in Figure 3. Tip cap 94 includes a body with an annular side wall 96 forming an axial bore or channel. Side wall 96 has an open end 98 having a dimension for receiving and coupling with tip 84 of syringe assembly 70. A flange 100 extends radially outward from open end 98 of side wall 96. Side wall 96 defines a cavity 102 for receiving tip 84 and cannula 90. As shown in Figure 3, cavity 102 has an axial length less than the length of cannula 90 so that the closed end of tip cap 94 engages the open end of cannula 90 to effectively close and seal syringe assembly 70. Typically, tip 92 of cannula 90 penetrates the end of tip cap 94 to seal the open end of cannula 90.

In the embodiments illustrated, the tip cap is shown as a unitary member made from a flexible and resilient polymeric material that exhibits low extractable content in the substance within the prefilled syringe. In alternative embodiments, the tip cap can have an outer cap made of a rigid plastic material that is coupled to a flexible and resilient inner cap. The outer rigid cap can include external threads for coupling with the internal threads of a luer collar to securely couple the tip cap to the end of the syringe barrel.

## Claims

1. A syringe tip cap (14) comprising:
a body (44) having a first open end (48), a closed second end (50) and a bore having an internal dimension for mating with a tip (24) of a syringe (10), **characterized in that** said body (44) being made from a polymer composition comprising a thermoplastic cyclic olefin polymer or copolymer and a thermoplastic elastomer and being sufficiently flexible to couple to and seal with the tip (24) of a syringe.

2. The syringe tip cap (14) of claim 1, wherein said thermoplastic cyclic olefin polymer or copolymer is a norbornene copolymer.

3. The syringe tip cap (14) of claim 1, wherein said body (44) is sterilizable by gamma radiation, ethylene oxide, autoclaving and combinations thereof without loss of flexibility and resilience and exhibits low extractables after sterilization.

4. The syringe tip cap (14) of claim 1, wherein said body (44) exhibits a residue upon ignition at 450°C-500°C of not more than 0.10 wt% based on the initial weight of said polymer composition.

5. The syringe tip cap (14) of claim 3, wherein after sterilization said body (44) exhibits low extractable levels as determined by 20 ml of a sample test solution having a residue of not more than 1.0 mg after evaporation and drying for 1 hour at 105°C, where said sample test solution is obtained by autoclaving said polymer composition in 200 ml of water at 121°C for 1 hour.

6. The syringe tip cap (14) of claim 1, wherein said polymer resin composition is sufficiently flexible and has elastomeric properties sufficient to couple with a frustoconical shaped syringe tip (24) or a threaded syringe tip (24).

7. The syringe tip cap (14) of claim 1, wherein said bore of said body (44) has an axial length complementing a length of a cannula (90) of said syringe, whereby a bottom end (50) of said bore is able to seal an open outer end of said cannula, and where said polymer resin composition is sufficiently flexible and has elastomeric properties sufficient to couple with a frustoconical shaped syringe tip (24).

8. The syringe tip cap (14) of claim 1, wherein said polymer composition comprises 20 wt% to 50 wt% of said thermoplastic elastomer.

9. The syringe tip cap (14) of claim 1, wherein said polymer composition comprises 30 wt% to 40 wt% of said thermoplastic elastomer.

10. The syringe tip cap (14) of claim 1, wherein said polymer composition consists essentially of norbornene cyclic olefin copolymer and said thermoplastic elastomer.

11. The syringe tip cap (14) of claim 1, wherein said thermoplastic elastomer is a styrene-ethylene-ethylene-propylene-styrene block copolymer.

12. The syringe tip cap (14) of claim 1, wherein said polymer composition comprises 50 wt% to 80 wt% of norbornene thermoplastic copolymer, and 20 wt % to 50 wt % of said thermoplastic elastomer.

13. A prefillable, low particle, low extractable syringe (10) comprising:
a syringe assembly (10) including a syringe barrel (12,72) having a substance receiving chamber (22) and a tip (24,84) extending from a distal end of said syringe barrel (12,72) and having a fluid passage extending through said tip (24,84), and a plunger (58) received in said syringe barrel (12,72), and
a tip cap (14,94) coupled to said tip (24,84) for closing said tip (24,84), said tip cap (14) being sufficiently flexible and resilient to be removably coupled to said tip (24,84) and form a substantially fluidtight seal with said tip (24,84), **characterized in that** said tip cap (14,94) being made from an elastic and flexible polymer composition comprising a thermoplastic cyclic olefin polymer or copolymer and a thermoplastic elastomer.

14. The prefillable syringe (10) of claim 13, wherein said syringe assembly (10) and tip cap (14) exhibit low extractables after sterilization by gamma radiation, ethylene oxide, autoclaving and combinations thereof as determined by extractables in solution as determined by a sample test solution having a residue of not more than 1.0 mg after evaporation and drying for 1 hour at 105°C, where said sample solution is obtained by autoclaving said polymer composition in 200 ml of water at 121°C for 1 hour.

15. The prefillable syringe (10) of claim 13, wherein said tip (24,84) of said syringe has a substantially frustoconical shape or a threaded tip (24,84) and where said tip cap (14,94) is sufficiently flexible and resilient after sterilization to couple with said tip (24) of said syringe and form said fluidtight seal.

16. The prefillable syringe (10) of claim 13, wherein said syringe barrel (12,72) has a luer collar (34) surrounding said top of said syringe barrel (12,72) and having internal threads, and where said tip cap (14,94) has external threads complementing said internal threads of said luer collar (34) for coupling said tip cap (14,94) to said syringe barrel (12,72).

17. The prefillable syringe (10) of claim 13, wherein said syringe (10) includes a cannula (90) extending from said tip (24,84) for delivering a substance from said syringe, and where said tip cap (14,94) has a channel with a closed end and an open end for coupling with said tip (24,84), said channel of said tip cap having an axial length complementing a length of said cannula (90) whereby an end of said cannula (90) penetrates a bottom end of said channel to seal an open end of said cannula (90).

18. The prefillable syringe (10) of claim 13, wherein said tip cap (14,94) exhibits a residue upon ignition at 450°C-500°C of not more than 0.10 wt% based on the initial weight of said polymer composition.

19. The prefillable syringe (10) of claim 13, wherein said polymer composition comprises 20 wt% to 50 wt% of said thermoplastic elastomer.

20. The prefillable syringe (10) of claim 13, wherein said polymer composition comprises 30 wt% to 40 wt% of said thermoplastic elastomer.

21. The prefillable syringe (10) of claim 13, wherein said polymer composition consists essentially of said thermoplastic cyclic olefin polymer and said thermoplastic elastomer.

22. The prefillable syringe (10) of claim 13, wherein said thermoplastic elastomer is a styrene-ethylene-ethylene-propylene-styrene block copolymer.

23. The prefillable syringe (10) of claim 13, wherein said thermoplastic cyclic olefin is a norbornene copolymer.

24. A prefilled syringe assembly (10) comprising:
a syringe barrel (12,72) having a hollow body with a first open end and a tip (14,84) with a bore extending into said hollow body;
a plunger (58) received in and closing said first open end;
a solution or suspension contained within said syringe barrel (12,72) to define said prefilled syringe; and
a tip cap (14,94) removably coupled to said tip (24,84), **characterized in that** said tip cap (14,94) being made from a flexible and resilient polymer composition comprising a thermoplastic cyclic olefin polymer or copolymer and a thermoplastic elastomer.

25. The prefilled syringe assembly (10) of claim 24, wherein said solution or suspension is a drug.

26. The prefilled syringe assembly (10) of claim 24, wherein said syringe assembly (10) exhibits low extractable levels after sterilization by gamma radiation, ethylene oxide, autoclaving or combinations thereof as determined by a 20 ml sample test solution having a residue of not more than 1.0 mg after evaporation and drying for 1 hour at 105°C, where said sample test solution is obtained by autoclaving said polymer composition in 200 ml of water at 121°C for 1 hour.

27. The prefilled syringe assembly (10) of claim 24, wherein said tip cap exhibits a residue upon ignition at 450°C-500°C of not more than 0.10 wt% based on the initial weight of said polymer composition.

28. The prefilled syringe assembly (10) of claim 24, wherein said thermoplastic cyclic olefin copolymer is a norbornene copolymer.

29. The prefilled syringe assembly (10) of claim 24, wherein said prefilled syringe assembly (10) is sterilizable by autoclaving and where said tip cap (14,94) remains sufficiently flexible to seal said tip (24,84) and exhibits low extractables after sterilization.

30. The prefilled syringe assembly (10) of claim 29, wherein said prefilled syringe assembly (10) is sterilizable by gamma radiation, ethylene oxide, autoclaving or combinations thereof and exhibits low extractables after sterilization.

31. The prefilled syringe assembly (10) of claim 24, wherein said polymer composition comprises 20 wt% to 50wt % of said thermoplastic elastomer based on the weight of said polymer composition.

32. The prefilled syringe assembly (10) of claim 24, wherein said polymer composition comprises 30 wt% to 40 wt% of said thermoplastic elastomer based on the weight of said polymer composition.

33. The prefilled syringe assembly (10) of claim 24, wherein said polymer composition consists essentially of said thermoplastic cyclic olefin copolymer and said thermoplastic elastomer.

34. The prefilled syringe assembly (10) of claim 24, wherein said thermoplastic cyclic olefin is a norbornene copolymer and where said polymer composition consists essentially of said norbornene copolymer and said thermoplastic elastomer.

## Patentansprüche

1. Spritzenspitzenkappe (14), umfassend:
einen Körper (44) mit einem ersten offenen Ende (48), einem geschlossenen zweiten Ende (50) und einer Bohrung mit einem Innenmaß zur Aufnahme einer Spitze (24) einer Spritze (10), **dadurch gekennzeichnet, dass** der Körper (44) aus einer Polymerzusammensetzung besteht, die ein thermoplastisches Polymer oder Copolymer aus einem cyclischen Olefin und ein thermoplastisches Elastomer umfasst und ausreichend biegsam ist, um an der Spitze (24) einer Spritze anzukoppeln und diese zu verschließen.

2. Spritzenspitzenkappe (14) nach Anspruch 1, wobei das thermoplastische Polymer oder Copolymer aus einem cyclischen Olefin ein Norbornen-Copolymer ist.

3. Spritzenspitzenkappe (14) nach Anspruch 1, wobei der Körper (44) durch Gammastrahlung, Ethylenoxid, Autoklavieren und Kombinationen davon ohne einen Verlust an Biegsamkeit und Rückprallelastizität sterilisierbar ist und nach der Sterilisierung einen niedrigen Gehalt an extrahierbaren Stoffen aufweist.

4. Spritzenspitzenkappe (14) nach Anspruch 1, wobei der Körper (44) einen Rückstand nach einem Glühen bei 450 °C - 500 °C von nicht mehr als 0,10 Gew.-%, bezogen auf das Anfangsgewicht der Polymerzusammensetzung, aufweist.

5. Spritzenspitzenkappe (14) nach Anspruch 3, wobei der Körper (44) nach der Sterilisation einen niedrigen Gehalt an extrahierbaren Stoffen, bestimmt mit 20 ml einer Probentestlösung mit einem Rückstand von nicht mehr als 1,0 mg nach der Verdampfung und einem 1-stündigen Trocknen bei 105 °C, aufweist, wobei die Probentestlösung erhalten wird, indem die Polymerzusammensetzung 1 h lang bei 121 °C in 200 ml Wasser autoklaviert wird.

6. Spritzenspitzenkappe (14) nach Anspruch 1, wobei die Polymerharz-Zusammensetzung ausreichend biegsam ist und Elastomereigenschaften hat, die ausreichend sind, um an eine kegelstumpfförmige Spritzenspitze (24) oder eine Gewinde-Spritzenspitze (24) anzukoppeln.

7. Spritzenspitzenkappe (14) nach Anspruch 1, wobei die Bohrung des Körpers (44) eine Axiallänge hat, die auf ein Stück einer Kanüle (90) der Spritze abgestimmt ist, wodurch ein unteres Ende (50) der Bohrung dazu fähig ist, ein offenes äußeres Ende der Kanüle zu verschließen und wobei die Polymerharz-Zusammensetzung ausreichend biegsam ist und elastomere Eigenschaften hat, die ausreichend sind, um an eine kegelstumpfförmige Spritzenspitze (24) anzukoppeln.

8. Spritzenspitzenkappe (14) nach Anspruch 1, wobei die Polymerzusammensetzung 20 Gew.-% bis 50 Gew.-% des thermoplastischen Elastomers umfasst.

9. Spritzenspitzenkappe (14) nach Anspruch 1, wobei die Polymerzusammensetzung 30 Gew.-% bis 40 Gew.-% des thermoplastischen Elastomers umfasst.

10. Spritzenspitzenkappe (14) nach Anspruch 1, wobei die Polymerzusammensetzung im Wesentlichen aus einem Norbornen-Copolymer eines cyclischen Olefins und dem thermoplastischen Elastomer besteht.

11. Spritzenspitzenkappe (14) nach Anspruch 1, wobei das thermoplastische Elastomer ein Styrol-Ethylen-Ethylen-Propylen-Styrol-Block-Copolymer ist.

12. Spritzenspitzenkappe (14) nach Anspruch 1, wobei die Polymerzusammensetzung 50 Gew.-% bis 80 Gew.-% eines thermoplastischen Norbornen-Copolymers und 20 Gew.-% bis 50 Gew.-% des thermoplastischen Elastomers umfasst.

13. Vorfüllbare Spritze (10) mit einem niedrigen Teilchengehalt und einem niedrigen Gehalt an extrahierbaren Stoffen, umfassend:
eine Spritze (10) einschließlich eines Spritzenzylinders (17, 72) mit einer Substanzaufnahmekammer (22) und einer Spitze (24, 84), die sich von einem distalen Ende des Spritzenzylinders (12, 72) erstreckt und einen Flüssigkeitsdurchlass aufweist, der sich durch die Spitze (24, 84) erstreckt, und einen Kolben (58), der vom Spritzenzylinder (12, 72) aufgenommen wird, und
eine Spitzenkappe (14, 94), die an die Spitze (24, 84) gekoppelt ist, um die Spitze (24, 84) zu schließen, wobei die Spitzenkappe (14) ausreichend biegsam und nachgiebig ist, um mit der Spitze (24, 84) entfernbar gekoppelt zu sein und eine im Wesentlichen flüssigkeitsdichte Dichtung mit der Spitze (14, 84) zu bilden, **dadurch gekennzeichnet, dass** die Spitzenkappe (14, 94) aus einer elastischen und biegsamen Polymerzusammensetzung besteht, die ein thermoplastisches Polymer oder Copolymer aus einem cyclischen Olefin und ein thermoplastisches Elastomer umfasst.

14. Vorfüllbare Spritze (10) nach Anspruch 13, wobei die Spritze (10) und die Spitzenkappe (14) nach einer Sterilisation mittels Gammastrahlung, Ethylenoxid, Autoklavieren und Kombinationen davon einen niedrigen Gehalt an extrahierbaren Stoffen aufweisen, wie durch die in einer Lösung extrahierbaren Stoffe mittels einer Probentestlösung mit einem Rückstand von nicht mehr als 1,0 mg nach einem Verdampfen und einem 1-stündigen Trocknen bei 105 °C bestimmt wird, wobei die Probentestlösung erhalten wird, indem die Polymerzusammensetzung 1 h lang bei 121 °C in 200 ml Wasser autoklaviert wird.

15. Vorfüllbare Spritze (10) nach Anspruch 13, wobei die Spitze (24, 84) der Spritze im Wesentlichen eine Kegelstumpfform oder eine Gewindespitze (24, 84) hat und wobei die Spitzenkappe (14, 94) nach der Sterilisation ausreichend biegsam und nachgiebig ist, um an die Spitze (24) der Spritze anzukoppeln und eine flüssigkeitsdichte Abdichtung zu ergeben.

16. Vorfüllbare Spritze (10) nach Anspruch 13, wobei der Spritzenzylinder (12, 72) einen Luer-Bund (34) hat, der die Oberseite des Spritzenzylinders (12, 72) umgibt und Innengewinde aufweist und wobei die Spitzenkappe (14, 94) Außengewinde aufweist, die komplementär zu den Innengewinden des Luer-Bundes (34) sind, um die Spitzenkappe (14, 94) an den Spritzenzylinder (12, 72) zu koppeln.

17. Vorfüllbare Spritze (10) nach Anspruch 13, wobei die Spritze (10) eine Kanüle (90) einschließt, die sich von der Spritze (24, 84) erstreckt, um eine Substanz aus der Spritze zu verabreichen, und wobei die Spitzenkappe (14, 94) einen Kanal mit einem geschlossenen Ende und einem offenen Ende zum Koppeln mit der Spitze (24, 84) aufweist, wobei der Kanal der Spitzenkappe eine Axiallänge hat, die auf ein Stück der Kanüle (90) abgestimmt ist, wodurch ein Ende der Kanüle (90) ein unteres Ende des Kanals so durchdringt, dass ein offenes Ende der Kanüle (90) verschlossen wird.

18. Vorfüllbare Spritze (10) nach Anspruch 13, wobei die Spitzenkappe (14, 94) einen Rückstand nach einem Glühen bei 450 °C - 500 °C von nicht mehr als 0,10 Gew.-%, bezogen auf das Anfangsgewicht der Polymerzusammensetzung, aufweist.

19. Vorfüllbare Spritze (10) nach Anspruch 13, wobei die Polymerzusammensetzung 20 Gew.-% bis 50 Gew.-% des thermoplastischen Polymers umfasst.

20. Vorfüllbare Spritze (10) nach Anspruch 13, wobei die Polymerzusammensetzung 30 Gew.-% bis 40 Gew.-% des thermoplastischen Polymers umfasst.

21. Vorfüllbare Spritze (10) nach Anspruch 13, wobei die Polymerzusammensetzung im Wesentlichen aus dem thermoplastischen Polymer aus einem cyclischen Olefin und dem thermoplastischen Polymer besteht.

22. Vorfüllbare Spritze (10) nach Anspruch 13, wobei das thermoplastische Elastomer ein Styrol-Ethylen-Ethylen-Propylen-Styrol-Block-Copolymer ist.

23. Vorfüllbare Spritze (10) nach Anspruch 13, wobei das thermoplastische cyclische Olefin ein Norbornen-Copolymer ist.

24. Vorgefüllte Spritze (10), umfassend:
einen Spritzenzylinder (12, 72) mit einem Hohlkörper mit einem ersten offenen Ende und einer Spitze (14, 84) mit einer Bohrung, die sich in den Hohlkörper erstreckt,
einen Kolben (58), der vom ersten offenen Ende aufgenommen wird und dieses verschließt,
eine Lösung oder Suspension, die im Spritzenzylinder (12, 72) enthalten ist, wodurch die vorgefüllte Spritze definiert ist, und
eine Spitzenkappe (14, 94), die mit der Spitze (24, 84) entfernbar gekoppelt ist, **dadurch gekennzeichnet, dass** die Spitzenkappe (14, 94) aus einer biegsamen und nachgiebigen Polymerzusammensetzung besteht, die ein thermoplastisches Polymer oder Copolymer aus einem cyclischen Olefin und ein thermoplastisches Elastomer umfasst.

25. Vorgefüllte Spritze (10) nach Anspruch 24, wobei die Lösung oder Suspension ein Medikament ist.

26. Vorgefüllte Spritze (10) nach Anspruch 24, wobei die Spritze (10) nach einer Sterilisation mittels Gammastrahlung, Ethylenoxid, Autoklavieren und Kombinationen davon einen niedrigen Gehalt an extrahierbaren Stoffen aufweist, wie durch die in einer Lösung extrahierbaren Stoffe mittels einer Probentestlösung mit einem Rückstand von nicht mehr als 1,0 mg nach einem Verdampfen und einem 1-stündigen Trocknen bei 105 °C bestimmt wird, wobei die Probentestlösung erhalten wird, indem die Polymerzusammensetzung 1 h lang bei 121 °C in 200 ml Wasser autoklaviert wird.

27. Vorgefüllte Spritze (10) nach Anspruch 24, wobei die Spitzenkappe einen Rückstand nach einem Glühen bei 450 °C - 500 °C von nicht mehr als 0,10 Gew.-%, bezogen auf das Anfangsgewicht der Polymerzusammensetzung, aufweist.

28. Vorgefüllte Spritze (10) nach Anspruch 24, wobei das thermoplastische Polymer oder Copolymer aus einem cyclischen Olefin ein Norbornen-Copolymer ist.

29. Vorgefüllte Spritze (10) nach Anspruch 24, wobei die vorgefüllte Spritze (10) durch Autoklavieren sterilisierbar ist und wobei die Spitzenkappe (14, 94) ausreichend biegsam bleibt, um die Spitze (24, 84) abzudichten und nach der Sterilisierung einen niedrigen Gehalt an extrahierbaren Stoffen aufweist.

30. Vorgefüllte Spritze (10) nach Anspruch 29, wobei die vorgefüllte Spritze (10) durch Gammastrahlung, Ethylenoxid, Autoklavieren und Kombinationen davon sterilisierbar ist und nach der Sterilisierung einen niedrigen Gehalt an extrahierbaren Stoffen aufweist.

31. Vorgefüllte Spritze (10) nach Anspruch 24, wobei die Polymerzusammensetzung 20 Gew.-% bis 50 Gew.-% des thermoplastischen Elastomers, bezogen auf das Gewicht der Polymerzusammensetzung, aufweist.

32. Vorgefüllte Spritze (10) nach Anspruch 24, wobei die Polymerzusammensetzung 30 Gew.-% bis 40 Gew.-% des thermoplastischen Elastomers, bezogen auf das Gewicht der Polymerzusammensetzung, aufweist.

33. Vorgefüllte Spritze (10) nach Anspruch 24, wobei die Polymerzusammensetzung im Wesentlichen aus dem thermoplastischen Copolymer des cyclischen Olefins und dem thermoplastischen Elastomer besteht.

34. Vorgefüllte Spritze (10) nach Anspruch 24, wobei das thermoplastische cyclische Olefin ein Norbornen-Copolymer ist und wobei die Polymerzusammensetzung im Wesentlichen aus dem Norbornen-Copolymer und dem thermoplastischen Elastomer besteht.

## Revendications

1. Capuchon d'embout de seringue (14), comprenant :
un corps (44) ayant une première extrémité ouverte (48), une deuxième extrémité fermée (50) et un alésage ayant une dimension interne pour s'adapter à un embout (24) d'une seringue (10), **caractérisé en ce que** ledit corps (44) est fait d'une composition polymère comprenant un polymère ou copolymère d'oléfine cyclique thermoplastique et un élastomère thermoplastique et **en ce qu'**il est suffisamment flexible pour se raccorder de manière étanche à l'embout (24) d'une seringue.

2. Capuchon d'embout de seringue (14) selon la revendication 1, dans lequel ledit polymère ou copolymère d'oléfine cyclique thermoplastique est un copolymère de norbornène.

3. Capuchon d'embout de seringue (14) selon la revendication 1, dans lequel ledit corps (44) peut être stérilisé par un rayonnement gamma, à l'oxyde d'éthylène, par autoclavage et par des combinaisons de ces procédés sans perte de souplesse ni de résilience, et présente après la stérilisation, un faible taux de matières extractibles.

4. Capuchon d'embout de seringue (14) selon la revendication 1, dans lequel ledit corps (44) présente un résidu à la calcination à une température allant de 450°C à 500°C non supérieur à 0,10% en poids, ramené au poids initial de ladite composition polymère.

5. Capuchon d'embout de seringue (14) selon la revendication 3, dans lequel, après la stérilisation, ledit corps (44) présente de faibles taux de matières extractibles, déterminés dans 20 ml d'une solution d'échantillon test ayant un résidu non supérieur à 1,0 mg après évaporation et séchage pendant 1 heure à 105°C, ladite solution d'échantillon test étant obtenue en autoclavant ladite composition polymère dans 200 ml d'eau à 121°C pendant 1 heure.

6. Capuchon d'embout de seringue (14) selon la revendication 1, dans lequel ladite composition de résine polymère est suffisamment flexible et a des propriétés élastomères suffisantes pour se raccorder à un embout de seringue de forme tronconique (24) ou à un embout de seringue fileté (24).

7. Capuchon d'embout de seringue (14) selon la revendication 1, dans lequel ledit alésage dudit corps (44) a une longueur axiale complétant une longueur d'une canule (90) de ladite seringue, de sorte qu'une extrémité inférieure (50) dudit alésage est capable d'obturer une extrémité extérieure ouverte de ladite canule, ladite composition de résine polymère étant suffisamment flexible et ayant des propriétés élastomères suffisantes pour se raccorder à un embout de seringue de forme tronconique (24).

8. Capuchon d'embout de seringue (14) selon la revendication 1, dans lequel ladite composition polymère comprend de 20% en poids à 50% en poids dudit élastomère thermoplastique.

9. Capuchon d'embout de seringue (14) selon la revendication 1, dans lequel ladite composition polymère comprend de 30% en poids à 40% en poids dudit élastomère thermoplastique.

10. Capuchon d'embout de seringue (14) selon la revendication 1, dans lequel ladite composition polymère est essentiellement constituée d'un copolymère de l'oléfine cyclique norbornène et dudit élastomère thermoplastique.

11. Capuchon d'embout de seringue (14) selon la revendication 1, dans lequel ledit élastomère thermoplastique est un copolymère à blocs de styrène/éthylène/éthylène/ propylène/styrène.

12. Capuchon d'embout de seringue (14) selon la revendication 1, dans lequel ladite composition polymère comprend de 50% en poids à 80% en poids de copolymère de norbornène thermoplastique et de 20% en poids à 50% en poids dudit élastomère thermoplastique.

13. Seringue préremplissable à faible taux de particules et de matières extractibles (10), comprenant :
un ensemble seringue (10) comprenant un fût de seringue (12,72) ayant une chambre destinée à recevoir une substance (22) et un embout (24,84) s'étendant depuis une extrémité distale dudit fût de seringue (12,72) et ayant un passage de fluide s'étendant à travers ledit embout (24,84) et un piston (58) logé dans ledit fût de seringue (12,72), et
un capuchon d'embout (14,94) raccordé audit embout (24,84) pour fermer ledit embout (24,84), ledit capuchon d'embout (14) étant suffisamment flexible et résilient pour se raccorder de manière amovible audit embout (24,84) et formant un joint pratiquement étanche aux fluides avec ledit embout (24,84),
**caractérisé en ce que** ledit capuchon d'embout (14,94) est fait d'une composition polymère résiliente et flexible comprenant un polymère ou un copolymère d'oléfine cyclique thermoplastique et un élastomère thermoplastique.

14. Seringue préremplissable (10) selon la revendication 13, dans laquelle ledit ensemble seringue (10) et le capuchon d'embout (14) présentent de faibles taux de matières extractibles après la stérilisation par un rayonnement gamma, à l'oxyde d'éthylène, par autoclavage et par des combinaisons de ces procédés, déterminés par les matières extractibles en solution mesurées dans une solution d'échantillon test ayant un résidu non supérieur à 1,0 mg après évaporation et séchage pendant 1 heure à 105°C, ladite solution d'échantillon étant obtenue par autoclavage de ladite composition polymère dans 200 ml d'eau à 121°C pendant 1 heure.

15. Seringue préremplissable (10) selon la revendication 13, dans laquelle ledit embout (24,84) de ladite seringue a une forme sensiblement tronconique ou un embout (24,84) fileté et dans laquelle ledit capuchon d'embout (14,94) est suffisamment flexible et résilient après la stérilisation pour se raccorder audit embout (24) de ladite seringue et former ledit joint étanche aux fluides.

16. Seringue préremplissable (10) selon la revendication 13, dans laquelle ledit fût de seringue (12,72) a une collerette luer (34) entourant ledit sommet dudit fût de seringue (12,72) et ayant des filetages internes, et dans laquelle ledit capuchon d'embout (14,94) a des filetages externes complémentaires desdits filetages internes de ladite collerette luer (34) pour raccorder ledit capuchon d'embout (14,94) audit fût de seringue (12,72).

17. Seringue préremplissable (10) selon la revendication 13, dans laquelle ladite seringue (10) comprend une canule (90) s'étendant depuis ledit embout (24,84) pour délivrer une substance à partir de ladite seringue et dans laquelle ledit capuchon d'embout (14,94) a un conduit avec une extrémité fermée et une extrémité ouverte pour se raccorder audit embout (24,84), ledit conduit dudit capuchon d'embout ayant une longueur axiale complémentaire d'une longueur de ladite canule (90), de sorte qu'une extrémité de ladite canule (90) pénètre dans une extrémité inférieure dudit conduit afin d'obturer une extrémité ouverte de ladite canule (90).

18. Seringue préremplissable (10) selon la revendication 13, dans laquelle ledit capuchon d'embout (14,94) présente un résidu à la calcination à une température allant de 450°C à 500°C non supérieur à 0,10% en poids, ramené au poids initial de ladite composition polymère.

19. Seringue préremplissable (10) selon la revendication 13, dans laquelle ladite composition polymère comprend de 20% en poids à 50% en poids dudit élastomère thermoplastique.

20. Seringue préremplissable (10) selon la revendication 13, dans laquelle ladite composition polymère comprend de 30% en poids à 40% en poids dudit élastomère thermoplastique.

21. Seringue préremplissable (10) selon la revendication 13, dans laquelle ladite composition polymère est essentiellement constituée dudit polymère d'oléfine cyclique thermoplastique et dudit élastomère thermoplastique.

22. Seringue préremplissable (10) selon la revendication 13, dans laquelle ledit élastomère thermoplastique est un copolymère à blocs de styrène/éthylène/éthylène/propylène/ styrène.

23. Seringue préremplissable (10) selon la revendication 13, dans laquelle ledit copolymère d'oléfine cyclique thermoplastique est un copolymère de norbornène.

24. Ensemble seringue préremplie (10), comprenant :
un fût de seringue (12,72) ayant un corps creux avec une première extrémité ouverte et un embout (14,84) avec un alésage s'étendant dans ledit creux corps ;
un piston (58) logé dans et fermant ladite première extrémité ouverte ;
une solution ou une suspension contenue dans ledit fût de seringue (12,72) pour définir ladite seringue préremplie ; et
un capuchon d'embout (14,94) raccordé de manière amovible audit embout (24,84), **caractérisé en ce que** ledit capuchon d'embout (14,94) est fait d'une composition polymère flexible et résiliente comprenant un polymère ou un copolymère d'oléfine cyclique thermoplastique et un élastomère thermoplastique.

25. Ensemble seringue préremplie (10) selon la revendication 24, dans lequel ladite solution ou suspension est un médicament.

26. Ensemble seringue préremplie (10) selon la revendication 24, dans lequel ledit ensemble seringue (10) présente de faibles taux de matières extractibles après la stérilisation par un rayonnement gamma, à l'oxyde d'éthylène, par autoclavage ou par des combinaisons de ces procédés, déterminés dans une solution d'échantillon test de 20 ml ayant un résidu non supérieur à 1,0 mg après évaporation et séchage pendant 1 heure à 105°C, ladite solution d'échantillon test étant obtenue par autoclavage de ladite composition polymère dans 200 ml d'eau à 121°C pendant 1 heure.

27. Ensemble seringue préremplie (10) selon la revendication 24, dans lequel ledit capuchon d'embout présente un résidu à la calcination à une température allant de 450°C à 500°C non supérieur à 0,10% en poids, ramené au poids initial de ladite composition polymère.

28. Ensemble seringue préremplie (10) selon la revendication 24, dans lequel ledit copolymère d'oléfine cyclique thermoplastique est un copolymère de norbornène.

29. Ensemble seringue préremplie (10) selon la revendication 24, dans lequel ledit ensemble seringue préremplie (10) peut être stérilisé par autoclavage et dans lequel ledit capuchon d'embout (14,94) reste suffisamment flexible pour obturer ledit capuchon (24,84) et présente de faibles taux de matières extractibles après la stérilisation.

30. Ensemble seringue préremplie (10) selon la revendication 29, dans lequel ledit ensemble seringue préremplie (10) peut être stérilisé par un rayonnement gamma, à l'oxyde d'éthylène, par autoclavage ou par des combinaisons de ces procédés et présente de faibles taux de matières extractibles après la stérilisation.

31. Ensemble seringue préremplie (10) selon la revendication 24, dans lequel ladite composition polymère comprend de 20% en poids à 50% en poids dudit élastomère thermoplastique, ramené au poids de ladite composition polymère.

32. Ensemble seringue préremplie (10) selon la revendication 24, dans lequel ladite composition polymère comprend de 30% en poids à 40% en poids dudit élastomère thermoplastique, ramené au poids de ladite composition polymère.

33. Ensemble seringue préremplie (10) selon la revendication 24, dans lequel ladite composition polymère est essentiellement constituée dudit copolymère d'oléfine cyclique thermoplastique et dudit élastomère thermoplastique.

34. Ensemble seringue préremplie (10) selon la revendication 24, dans lequel ledit copolymère d'oléfine cyclique thermoplastique est un copolymère de norbornène et dans lequel ladite composition polymère est essentiellement constituée dudit copolymère de norbornène et dudit élastomère thermoplastique.
